# EUROPEAN PATENT APPLICATION

(11) **EP 2 589 662 A1**
(43) Date of publication of application: **08.05.2013**
(21) Application number: 11801029.7
(22) Date of filing: 30.06.2011
(51) Int. Cl.: C12P 7/64, C12P 1/00

(54) **PROCESS FOR PRODUCTION OF USEFUL SUBSTANCE**

(30) Priority: 30.06.2010 JP 2010149001
(71) Applicant: Nippon Suisan Kaisha, Ltd., Chiyoda-ku Tokyo 100-8686 (JP)
(72) Inventor: KAMADA, Nozomu, Mishima-gun Osaka 6180012 (JP)
(74) Representative: Wallinger, Michael
(86) International application number: PCT/JP2011/065524
(87) International publication number: WO 2012/002572

(57) **Abstract**

The present invention relates to a processd for producing a useful substance using microorganisms, and an object thereof is to provide a method that shortens the culturing period of the microorganisms and improves the productivity of the useful substance.

The speed at which microorganisms consume carbohydrates and/or hydrocarbon oxidation products can be increased and the productivity of a target useful substance can be improved by using a main culture initial medium in which the total concentration of carbohydrate and hydrocarbon oxidation product is less than 0.4% by weight in terms of the carbon equivalent.

## Description

### TECHNICAL FIELD

The present invention relates to a production method of useful substances that utilizes microorganisms.

### BACKGROUND ART

Well-known examples of processes used to produce useful substances by utilizing microorganisms include the production of L-amino acids, nucleic acids, unsaturated fatty acids etc. by fermentation (Non-Patent Documents 1 and 2). These useful substances are produced using the metabolism of microorganisms and using carbohydrates such as glucose and/or hydrocarbon oxidation products as substrates. Known examples of processes used to culture microorganisms for producing useful substances include batch culturing wherein all culture medium components including substrates are added in an initial culture and, fed-batch culturing wherein culturing is started with an initial medium to which a portion of substrates is added and inoculated with microorganism followed by addition of remaining substrates to the medium either intermittently or continuously, and continuous culturing wherein a fresh medium containing substrates is added to a fermentation vessel at a constant rate, and then culturing is continued while discharging an equal amount of culture broth outside the tank. However, in any of these culturing methods, carbohydrates such as glucose or starch-saccharifying products and/or hydrocarbon oxidation products serving as substrates of useful substances are contained in a medium for industrially producing useful substances at the time the medium is inoculated with microorganisms. There is currently no known method for starting culturing of microorganisms without adding a carbohydrates such as glucose or starch-saccharifying products and/or hydrocarbon oxidation product to the initial medium.

### Prior Art Documents

### Non-Patent Documents

Non-Patent Document 1: Applied Microbiology, Revised Edition, Baifukan Publishing Co., Ltd., 1993
Non-Patent Document 2: Science and Engineering, Vol. 74, No. 1, 26 (2000)

### DISCLOSURE OF THE INVENTION

### Problems to be Solved by the Invention

An object of the present invention is to provide a process for efficiently producing useful target substances by utilizing microorganisms, and more particularly, to provide a process for enhancing the production efficiency of useful substances while shortening a culturing period required to produce the useful substances.

### Means for Solving the Problems

As a result of conducting extensive studies to solve the aforementioned problems, the inventors of the present invention found that, in a process for producing useful substances by culturing microorganisms, useful substances can be efficiently produced by using an initial medium for main culturing in which the total concentration of carbohydrates and hydrocarbon oxidation products is less than 0.4% by weight in terms of the carbon equivalent, and then adding carbohydrates and/or hydrocarbon oxidation products after the microorganisms have begun logarithmic growth, thereby leading to completion of the present invention.

Namely, the present includes, but is not limited to, the inventions indicated below.
(1) A process for producing a useful substance by culturing microorganisms, comprising: a pre-culturing step for pre-culturing a microorganism; a step for inoculating the resulting pre-culture broth into a main culture initial medium, wherein the total concentration of carbohydrate and hydrocarbon oxidation product in the main culture initial medium is less than 0.4% by weight in terms of the carbon equivalent; and a main culturing step for adding hydrocarbon and/or hydrocarbon oxidation product after the microorganisms have begun logarithmic growth.
(2) The process for production according to the above (1), wherein the carbohydrate and/or hydrocarbon oxidation product is substantially not added to the main culture initial medium.
(3) The process for production according to the above (1) or (2), wherein the carbohydrate and/or hydrocarbon oxidation product added after the microorganisms have begun logarithmic growth is a sugar.
(4) The process for production according to any one of the above (1) to (3), wherein the microorganism is a microorganism that catabolize the carbohydrate and/or hydrocarbon oxidation product with an exoamylase.
(5) The process for production according to any one of the above (1) to (4), wherein the microorganism is a microorganism belonging to the genus *Mortierella.*
(6) The process for production according to any one of the above (1) to (5), wherein the useful substance is a highly unsaturated fatty acid.

### Effects of the Invention

According to the present invention, a useful substance can be efficiently produced by a microorganism.

### BRIEF DESCRIPTION OF THE DRAWINGS

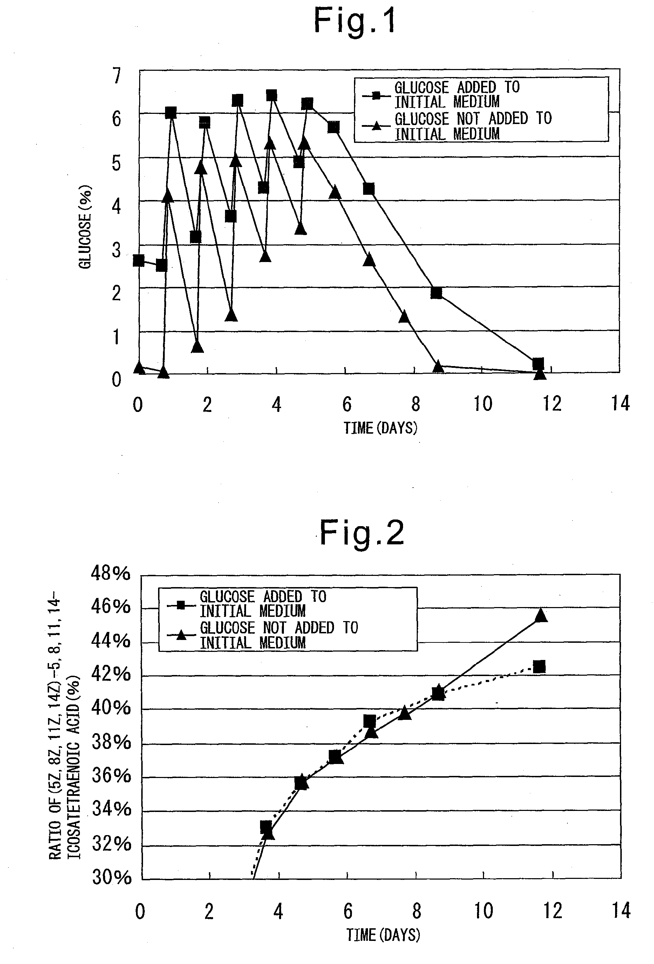

FIG. 1 is a graph indicating change in glucose concentration in an experiment for production of (5Z,8Z,11Z,14Z)- 5,8,11,14-icosatetraenoic acid in Example 1, in a culture system in which glucose was added to an initial medium and a culture system in which glucose was not added to an initial medium.
FIG. 2 is a graph indicating change in the ratio of 5Z,8Z,11Z,14Z)-5,8,11,14-icosatetraenoic acid to total fatty acids in an experiment for production of 5Z,8Z,11Z,14Z)-5,8,11,14-icosatetraenoic acid in Example 1, in a culture system in which glucose was added to an initial medium and a culture system in which glucose was not added to an initial medium in Example 1.

### EMBODIMENTS FOR CARRYING OUT THE INVENTION

As described above, the present invention relates to a process for producing a useful substance by culturing a microorganism, comprising: a pre-culturing step for pre-culturing the microorganisms; a step for inoculating the resulting microorganism into a main culture initial medium, wherein the total concentration of carbohydrate and hydrocarbon oxidation product in the main culture initial medium is less than 0.4% by weight in terms of the carbon equivalent; and a main culturing step for adding a carbohydrate and/or hydrocarbon oxidation product after the microorganism has begun logarithmic growth.

There are no particular limitations on the useful substances to be produced according to the present invention provided they are substances that can be produced by microorganisms, and are exemplified by amino acids, nucleic acids, unsaturated fatty acids, lipids, proteins, peptides, vitamins, sugars, sugar-alcohols, alcohols, organic acids, antibiotics and physiologically active substances etc.

Examples of amino acids include L-glutamic acid, L-lysine, L-threonine, L-tryptophan, L-phenylalanine, L-tyrosine, L-leucine, L-isoleucine, L-valine, L-asparagine, L-histidine, L-glutamine, L-arginine, L-ornithine, L-citrulline, L-proline, L-serine, methionine, L-alanine, L-cysteine, cystine and homoserine, while examples of nucleic acids include inosine, guanosine, inosinic acid, guanylic acid, xanthylic acid and cytidylic acid.

Examples of unsaturated fatty acids include (6Z,9Z,12Z)-6,9,12-octadecatrienoic acid, (8Z,11Z,14Z)-8,11,14-icosatrienoic acid, (5Z,8Z,11Z,14Z)-5,8,11,14-icosatetraenoic acid, (4Z,7Z,11Z,14Z,17Z)-4,7,11,14,17-docosapentaenoic acid,' (9Z, 12Z, 15Z) -9, 12, 15-octadecatrienoic acid, (6Z,9Z,12Z,15Z)-6,9,12,15-octadecatetraenoic acid, (8Z,11Z,14Z,17Z)-8,11,14,17-eicosatetraenoic acid, (5Z,8Z,11Z,14Z,17Z)-5,8,11,14,17-eicosapentaenoic acid, (4Z,7Z,10Z,13Z,16Z,19Z)-4,7,10,13,16,19-docosahexaenoic acid, (6Z,9Z)-6,9-octadecadienoic acid, (8Z,11Z)-8,11-icosadienoic acid, (5Z,8Z,11Z)-5,8,11-icosatrienoic acid, etc, while examples of lipids include triacylglycerides, diacylglycerides, monoacylglycerides, phosphatidyl choline, phosphatidyl serine, phosphatidyl ethanolamine, phosphatidyl inositol, phosphatidic acid, lysophosphatidyl choline, lysophosphatidyl serine, lysophosphatidyl ethanolamine, lysophosphatidyl inositol, lysophosphatidic acid, cardiolipin, sterols etc containing the aforementioned unsaturated fatty acids.

Examples of proteins include lipases, phospholipases, proteases, transglutaminases, amylases, isoamylases, glucose isomerases, glucosidases, pullulanases, polygalacturonases, mannose isomerases, arabinofuraosylases, rhamnosydases, mannosydases, cyclodextrin-producing enzymes, glucose dehydrogenases, glucose-6-phosphate dehydrogenases, hexokinases, glucose oxidases, sialyltransferases, fucosyltransferases, N-acetylglucosaminyltransferases, galactosyltransferases, mannosyltransferases, N-acetylgalactosaminidases, N-acylamino acid racemases, D-aminoacylases, L-phenylserine dehydrogenases, carbonyl reductases, α-keto acid reductases, peroxidases, laccases, cholesterol oxidases, fructosyl amino acid oxidases, manganese peroxidases, ascorbic acid oxidases, glucose oxidases, nitrilases, nitrile hydralases, phytases, fumarases, uricases, catalases, phosphatases, ureases, cellulases, invertases, asparaginases, tannases, lactases, urokinase etc, while examples of peptides include glutathione.

Examples of vitamins include ascorbic acid, riboflavin, thiamine, niacin, biotin, cyanocobalamin, vitamin A, vitamin D, vitamin K and carotenoids etc, examples of sugars include glucosamine, N-acetylglucosamine, xylose, ribose, fructooligosaccharides, galactooligosaccharides, mananooligosaccharides, chitins, chitosans, hyaluronic acid, viscous polysaccharide etc, and examples of sugar-alcohols include xylitol, erythritol, galactitol, mannitol, sorbitol etc.

Examples of alcohols include ethanol, butanol and glycerol, examples of organic acids include acetic acid, lactic acid, pyruvic acid, succinic acid, citric acid, kojic acid, fumaric acid, malic acid, gluconic acid, itaconic acid, formic acid, propionic acid, butyric acid, isobutyric acid, acrylic acid, methacrylic acid, sorbic acid, maleic acid, cinnamic acid, aldonic acid, tartaric acid, isocitric acid, ketoaldonic acid, 2-ketogulonic acid etc, and examples of antibiotics include benzoquinone-based antibiotics, anthracycline-based antibiotics etc.

Examples of physiologically active substances include nicotinamide adenine dinucleotide, flavin adenine dinucleotide, orotic acid, shikimic acid, folic acid, hydroxycitric acid, cerebroside, astaxanthin, S-adenosylmethionine, S-methylmethionine sulfonium chloride, retinoids, cerebroside, coenzyme A, coenzyme Q10, inositol, choline, carnitine, pyrroloquinolinequinone, gibberellin, abscisic acid, polyhydroxyalkanoate, cyclosporin A, mevalotin, polyglutamic acid, polylysine, mannosylerythritol lipid etc.

Preferably, examples of unsaturated fatty acids include (6Z,9Z,12Z)-6,9,12-octadecatrienoic acid, (8Z,11Z,14Z)-8,11,14-icosatrienoic acid, (5Z,8Z,11Z,14Z)-5,8,11,14-icosatetraenoic acid, (4Z,7Z,11Z,14Z,17Z)-4,7,11,14,17-docosapentaenoic acid, (9Z,12Z,15Z)-9,12,15-octadecatrienoic acid, (6Z,9Z,12Z,15Z)-6,9,12,15-octadecatetraenoic acid, (8Z,11Z,14Z,17Z)-8,11,14,17-eicosatetraenoic acid, (5Z,8Z,11Z,14Z,17Z)-5,8,11,14,17-eicosapentaenoic acid, (4Z,7Z,10Z,13Z,16Z,19Z)-4,7,10,13,16,19-docosahexaenoic acid, (6Z, 9Z) -6, 9-octadecadienoic acid, (8Z, 11Z)-8,11-icosadienoic acid, (5Z,8Z,11Z)-5,8,11-icosatrienoic acid etc, and examples of lipids include triacylglycerides, diacylglycerides, monoacylglycerides, phosphatidyl choline, phosphatidyl serine, phosphatidyl ethanolamine, phosphatidyl inositol, phosphatidic acid, lysophosphatidyl choline, lysophosphatidyl serine, lysophosphatidyl ethanolamine, lysophosphatidyl inositol, lysophosphatidic acid, cardiolipin, sterols etc containing the aforementioned unsaturated fatty acids.

More preferably, examples of unsaturated fatty acids include (8Z,11Z,14Z)-8,11,14-icosatrienoic acid, (5Z,8Z,11Z,14Z)-5,8,11,14-icosatetraenoic acid, (5Z,8Z,11Z)-5,8,11-icosatrienoic acid etc, and examples of lipids include triacylglycerides, diacylglycerides, monoacylglycerides, phosphatidyl choline, phosphatidyl serine, phosphatidyl ethanolamine, phosphatidyl inositol, phosphatidic acid, lysophosphatidyl choline, lysophosphatidyl serine, lysophosphatidyl ethanolamine, lysophosphatidyl inositol, lysophosphatidic acid etc.

The process of the present invention can be applied even to substances currently having not be produced using microorganisms but can be produced using microorganisms. Useful substances produced by microorganisms may present alone or may be a mixture of a plurality of two or more useful substances. In addition, the useful substance may be a substance that contains the aforementioned useful substances as constituents thereof.

There are no particular limitations on the microorganisms used in the present invention, and any microorganisms can be used provided they are microorganisms useful to produce useful substances by fermentation. In addition, the process of the present invention can also be applied to microorganisms previously not used to produce useful substances by fermentation provided they have the ability to produce useful substances. In addition, microorganisms inherently not having the ability to produce useful substances can also be used in the process of the present invention after they acquire the ability to produce useful substances as a result of breeding using a method such as mutation or recombinant DNA technology known among persons with ordinary skill in the art.

Specific examples of microorganisms used in the present invention include microorganisms belonging to the genera *Escherichia, Corynebacterium, Bacillus, Serratia, Erwinia, Pseudomonas, Rhodobacter, Salmonella, Vibrio, Xanthomonas, Desulfurococcales, Thermococcus, Arthrobacter, Enterobacter, Acetobacterium, Acetobacter, Methanothrix, Agrobacterium, Zoogloea, Alcaligenes, Enterococcus, Deinococcus, Sebekia, Cellulomonas, Cryptococcus, Acinetobacter, Geobacillus, Natrialba, Exiguobacterium, Sphingomonas, Thermobifida, Leuconostoc, Lactobacillus, Acidiphilium, Nocardiopsis, Streptomyces, Amycolata, Pseudonocardia, Thermomonospora, Streptobacterium, Gordonia, Amycolatopsis, Pyrococcus, Thermococcus, Thermoplasma, Thermus, Pediococcus, Saccharomyces, Schizosaccharomyces, Candida, Torulaspora, Kluyveromyces, Pichia, Pseudozyma, Debaryomyces, Pachysolen, Mortierella,* subgenus *Mortierella, Conidiobolus, Pythium, Phytophthora, Cladosporium, Rhodotorula, Entomophthora, Echinosporangium, Saprolegnia, Ulkenia, Schizochytrium, Thraustochytrium, Mortiella, Trichoderma, Aspergillus, Rhizopus, Acremonium, Penicillium, Phoma, Myrothecium, Trametes, Hypomyces, Fusarium, Auricularia, Faecium, Menisporopsis, Thermomyces, Cercospora, Botrytis, Stemphylium, Monascus, Mucor, Pestalotia, Phlebia, Schizophyllum, Chloroflexus, Chytasporicia, Chrysosporium* etc, and there are no particular restrictions thereon provided they are microorganisms used to produce useful substances.

The following lists more specific examples of microorganisms. For example, in the case the useful substance to be produced is an unsaturated fatty acid or lipid, a filamentous fungus of the genus *Mortierella* is preferable, a filamentous fungus of the subgenus *Mortierella* is more preferable, and *Mortierella alpina* is particularly preferable. For example, microorganisms belonging to the genera *Mortierella, Conidiobolus, Pythium, Phytophthora, Penicillium, Cladosporium, Mucor, Fusarium, Aspergillus, Rhodotorula, Entomophthora, Echinosporangium* or *Saprolegnia* can be used as microorganisms having the ability to produce a lipid containing as a constituent fatty acid thereof (5Z,8Z,11Z,14Z)-5,8,11,14-icosatetraenoic acid.

More specifically, examples of microorganisms belonging to the genus *Mortierella*, subgenus *Mortierella* include *Mortierella elongata, Mortierella exigua, Mortierella hygrophila, Mortierella alpina* etc. Specific examples of strains thereof include *Mortierella elongate* IFO8570, *Mortierella exigua* IF08571, *Mortierella hygrophila* IF05941 and *Mortierella alpina* IF08568, ATCC16266, ATCC32221, ATCC32222, ATCC42430, CBS219.35, CBS224.37, CBS250.53, CBS343.66, CBS527.72, CBS529.72, CBS608.70 and CBS754.68, etc.

All of these strains can be acquired from the Biological Research Center of the National Institute of Technology and Evaluation (NBRC), American Type Culture Collection (ATCC) or Centraal Bureau voor Schimmelcultures (CBS). In addition, in the case of producing (5Z,8Z,11Z,14Z)-5,8,11,14-icosatetraenoic acid or (5Z,8Z,11Z,14Z)-5,8,11,14-icosatetraenoic acid-containing lipid, *Mortierella alpina* strain IS-4 or *Mortierella elongata* strain SAM0129 (FERM P-8703) (FERM BP-1239) can be used. In the case of producing (8Z,11Z,14Z)-8,11,14-icosatrienoic acid or (8Z,11Z,14Z)-8,11,14-icosatrienoic acid-containing lipid, *Mortierella alpina* strain SAM1860 (FERM BP-3589), *Mortierella alpina* strain S14, *Mortierella alpina* strain Iz3 etc can be used, which are mutant strains deficient in Δ5 desaturation activity that were obtained by deriving from strain IS-4 using a conventional procedure, i.e., nitrosoguanidine mutation. In the case of producing (5Z,8Z,11Z)-5,8,11-icosatrienoic acid or (5Z,8Z,11Z)-5,8,11-icosatrienoic acid-containing lipid, *Mortierella alpina* strain SAM1861 (FERM BP-3590), *Mortierella alpina* strain M209-7 (FERM P-15766) or *Mortierella alpina* strain JT180 can be used, which are mutant strains deficient in Δ12 desaturation activity that were obtained by deriving from strain IS-4 using a conventional procedure, i.e., nitrosoguanidine mutation.

A microorganism is pre-cultured in a medium suitable for the microorganism under conditions that allow obtaining of microorganism required for main culturing.
Inoculation of the resulting microorganism into a main culture initial medium may be suitably selected by a person with ordinary skill in the art depending of a culturing method. More specifically, vegetative cells, spores and/or mycelia of a microbial strain, or a seed culture obtained by pre-culturing the microbial strain, or vegetative cells, spores and/or mycelia recovered from a seed culture obtained by pre-culturing the microbial strain, are inoculated into a liquid culture or solid culture followed by culturing.

Examples of carbohydrates or hydrocarbon oxidation products that can be used in a main culture medium include, but are not limited to, glucose, fructose, xylose, saccharose (sucrose), maltose, soluble starch, molasses, glycerol, mannitol, sorbitol, galactose or saccharifycation products of starch and the like either alone or in combination, as well as citrus molasses, beet molasses, beat juice or sugar cane juice and the like containing these, substances able to be assimilated as carbon sources by the microorganisms, and substances typically used by persons with ordinary skill in the art. The initial total concentration of carbohydrates and hydrocarbon oxidation products, in terms of the carbon equivalent, is less than 0.4% by weight, preferably 0.2% by weight or less, more preferably 0.1% by weight or less, even more preferably 0.05% by weight or less and particularly preferably 0% by weight, or in other words, carbohydrates and/or hydrocarbon oxidation products are substantially not added to the initial medium. Here, less than 0.4% by weight in terms of the carbon equivalent refers to 1% by weight in the case of glucose, 0.95% by weight in the case of sucrose, 0.77% by weight in the case of ethanol, and 1.02% by weight in the case of glycerol. Carbohydrates and/or hydrocarbon oxidation products transferred from pre-culturing are usually minor, and are equivalent to "carbohydrates and/or hydrocarbon oxidation products substantially not added to the main culture initial medium". In addition, the addition of carbohydrates and/or hydrocarbon oxidation products that are not expected to be metabolized and utilized by the microorganisms is also equivalent to "carbohydrates and/or hydrocarbon oxidation products are substantially not added to the main culture initial medium".

Carbohydrates and/or hydrocarbon oxidation products are added to a main culture initial medium after a pre-culture broth is inoculated into the main culture initial medium, and preferably after completion of the lag phase of the microorganisms and after entering into the log phase. Although the lag phase and log phase of the microorganisms vary dependent on the microorganisms used for fermentative production of the useful substance, the medium, culturing conditions and the like, a person with ordinary skill in the art is able confirm the lag phase and log phase of the microorganisms by measuring the cell count, turbidity of the culture broth, or dry or wet cell weight per culture broth and the like using a known method, or by measuring a pH value or dissolved oxygen concentration of the culture broth or a carbon dioxide concentration present in gas exhausted from the culturing vessel, and confirming changes in each parameter. For example, in the case of using cell count for the parameter, a time at which the cell count begins to increase exponentially is the time when the microorganisms shift from the lag phase to the log phase. In addition, in the case of investigating dissolved oxygen concentration, a time when oxygen consumption begins to increase exponentially is the time when the microorganisms shift from the lag phase to the log phase.

The number of times for adding carbohydrates and/or hydrocarbon oxidation products may be one time or a plurality of times, and the carbohydrates and/or hydrocarbon oxidation products can be added to the medium either continuously or intermittently. There are no particular limitations on the total amount of carbohydrates and/or hydrocarbon oxidation products to be added.

Nitrogen sources are those typically used to culture microorganisms, and include natural nitrogen sources such as peptones, yeast extract, wheat germ extract, beef extract, casamino acid, corn steep liquor, soybean protein, defatted soybeans, cottonseed meal, casein hydrolysates, various types of fermentation cells and digestion products thereof, and inorganic nitrogen sources such as sodium nitrate, ammonium nitrate and other nitrates, ammonium sulfate, ammonium chloride or ammonium phosphate, and they can be used alone or in combination thereof.

If necessary, other substances can also be used, such as trace nutrients, examples of which include phosphate ions, potassium ions, sodium ions, magnesium ions, calcium ions, iron, copper, zinc, manganese, nickel, cobalt and other metal ions, as well as vitamins such as biotin or thiamine. Antifoaming agents such as Adekanate or silicone can also be added as necessary.

An existing method used to culture microorganisms can be used for main culturing, specific examples of which include solid culturing, static culturing, shake culturing and aeration-agitation culturing. Examples of culturing procedures that can be used include so-called batch culturing, fed-batch culturing, repetitive batch culturing and continuous culturing. Furthermore, an impeller (stirring blade), air lift fermentation tank, fermentation broth pump-driven circulator or a combination of these means can be used as agitation means.

Although the production process of the present invention can be carried out with any of the aforementioned culturing methods, it is industrially advantageous to carry out main culturing by aeration-agitation culturing in a liquid medium in particular. Although a fermentation vessel such as jar fermenter or tank can be used to carry out aeration-agitation culturing, the fermentation vessel is not limited thereto.

In aeration-agitation culturing, the culture medium may be aerated with a gas containing oxygen such as air, or a gas not containing oxygen, such as argon or nitrogen, or the medium may be aerated with a mixed gas thereof, and this gas is suitably selected dependent on the conditions of the culturing system.

Although varying dependent on a microorganism used, the culturing temperature is normally 5°C to 100°C, preferably 10°C to 50°C and more preferably 15°C to 40°C. In addition, as one embodiment thereof, the microorganism is cultured at a temperature suitable for microbial growth, followed by continuing culturing at a temperature suitable for production of a target substance to enhance productivity of the target substance.

The pre-culturing period in a solid medium or in a liquid medium in a test tube or flask is normally 5 hours to 10 days, preferably 5 hours to 5 days and more preferably 5 hours to 3 days. The subsequent main culturing period for producing a useful substance is normally 5 hours to 30 days, preferably 5 hours to 20 days and more preferably 5 hours to 15 days. The amount of a desired useful substance produced, the amount of nutrients in the medium or the residual amount of substrate component can be used as criteria for determining the time of completion of main culturing.

Furthermore, the pH value of the medium used to culture the microorganisms is 3 to 9, and pH can be adjusted using an inorganic or organic acid, alkaline solution, urea, calcium carbonate or ammonia and the like.
A useful substance is allowed to accumulate within microbial cells, on the surface of microorganisms or in the medium by culturing the microorganisms in this manner. Although the culture broth containing microbial cells per se or a concentrate thereof can be used as the final target substance, a useful substance can also be recovered and purified as necessary. In the case the target useful substance accumulates within or on the surface of microbial cells, the microbial cells can be recovered from the culture broth by a conventional method such as centrifugal separation or filtration, and the microbial cells per se can then be used as the final target substance, or the target substance can be recovered with a solvent and the like, and the target substance can be purified using a conventional method used to purify target substances, such as ion exchange chromatography, gel filtration chromatography, adsorption chromatography, thin layer chromatography, high-performance liquid chromatography or crystallization. In addition, in the case a target substance accumulates in the medium, although the culture broth per se, from which microbial cells have been removed, or a concentrate thereof can be used as a target substance, the target substance can also be purified using a conventional purification method as described above.

Examples of microorganisms that produce useful substances in the form of unsaturated fatty acids or lipids include microorganisms belonging to the genera *Mortierella, Conidiobolus, Pythium, Phytophthora, Penicillium, Cladosporium, Mucor, Fusarium, Aspergillus, Rhodotorula, Entomophthora, Echinosporangium* and *Saprolegnia,* and these microorganisms accumulate useful substances in the form of unsaturated fatty acids or lipids within their cells.

In particular, since microorganisms that catabolize carbohydrates and/or hydrocarbon oxidation products by exoamylase have a low level of activity for decomposing high molecular weight α-glycans represented by starch, and thus metabolically catabolize carbohydrates and/or hydrocarbon oxidation products at a slow rate, carbohydrates and/or hydrocarbon oxidation products are substantially not contained in the main culture initial medium, thereby making this preferable.

Microorganisms that catabolize these carbohydrates and/or hydrocarbon oxidation products by exoamylase are particularly preferable, because such microorganisms are able to immediately react with sugar in the case where the carbohydrate and/or hydrocarbon oxidation product added after the microorganisms have begun logarithmic growth is a sugar.

Following completion of culturing, unsaturated fatty acids, lipids containing unsaturated fatty acids or microbial cells containing them can be obtained from the medium according to a method well known among persons with ordinary skill in the art, such as by referring to the method described in Japanese Unexamined Patent Publication No. 2000-69987.

The microbial cells are preferably dried after sterilizing as desired. Drying can be carried out by heating in an oven, freeze-drying or hot air drying and the like. Lipids containing unsaturated fatty acids can be obtained from the dry or wet cells using a method well known among persons with ordinary skill in the art. For example, a lipid containing a high concentration of unsaturated fatty acid contained mainly in triglyceride is obtained by extracting dry cells with an organic solvent such as hexane followed by distilling off the organic solvent from the extract under reduced pressure. In addition, a highly pure refined edible oil (triglyceride) can be obtained by purifying the lipid consisting mainly of triglyceride using an ordinary purification treatment carried out on edible fats and oils such as degumming, deacidification, decolorization or deodorization.

Although unsaturated fatty acids present in lipids can be separated directly, they can be easily separated from other lipid components in a form of an ester of a lower alcohol such as in form of methyl ester. In addition, a desired unsaturated fatty acid can be easily separated from other unsaturated fatty acids. These types of separation procedures are well known among persons with ordinary skill in the art.

Microbial cells of the present invention can be used as is or used after drying. In addition, unsaturated fatty acids and lipids containing them obtained with the production process of the present invention can also be used in various ways. These can be used in, for example, dietary supplements, nutritional compositions, animal feeds (and particularly pet foods), fish farming feeds or powdered milk using a method well known among persons with ordinary skill in the art.

Although the following indicates Examples of the present invention, the present invention is not limited to the following examples.

### Example 1

### <1> Production of (5Z,8Z,11Z,14Z)-5,8,11,14-icosatetraenoic Acid Using Culturing System in which Glucose was Added to Initial Medium

*Mortierella alpina* strain 1S-4 was used. 100 µL of frozen spore fluid were inoculated into 100 mL of medium at pH 6.3 containing 1.0 w/w% of yeast extract and 2.0 w/w% of aqueous glucose followed by starting pre-culturing (first stage) under conditions of reciprocally shaking at 100 rpm at a temperature of 28°C, and culturing for 3 days. Next, 30 L of a medium having a pH of 6.3 and containing 1.0 w/w% of yeast extract, 2.0 w/w% of aqueous glucose and 0.1 w/w% of soybean oil was prepared in a 50 L fermentation vessel, and 200 mL of the seed culture broth (first stage) were inoculated therein, followed by starting pre-culturing (second stage) and culturing for 2 days under conditions of a temperature of 28°C. Next, 25 L of the seed culture broth (second stage) was inoculated into a main culture initial medium (2.67 w/w% of aqueous glucose, 5.0 w/w% of defatted soybean powder, 0.3 w/w% of K₂HPO₄, 0.05 w/w% of MgCl₂·₆H₂O, 0.05 w/w% of CaCl₂·2H₂0, pH 6.0), and brought to a total of 4000 L for the amount of the initial culture medium.

Culturing was started at a temperature of 26°C and internal pressure of 0.10 MPa. Aqueous glucose was added at 5.33% on day 1 of culturing, at 5.33% on day 2, at 4.00% on day 3, at 4.00% on day 4 and 2.67% on day 5, and as a result of adding aqueous glucose at a total of 24.0%, glucose in the medium was completely depleted on day 12 of culturing. The amount of (5Z,8Z,11Z,14Z)-5,8,11,14-icosatetraenoic acid produced on day 12 of culturing was 17.7 g/L, and the ratio of (5Z,8Z,11Z,14Z)-5,8,11,14-icosatetraenoic acid to the total amount of fatty acid was 42.4%.

### <2> Production of (5Z,8Z,11Z,14Z)-5,8,11,14-icosatetraenoic Acid Using Culturing System in which Glucose was not Added to Initial Medium

*Mortierella alpina* strain 1S-4 was used. 100 µL of frozen spore fluid was inoculated into 100 mL of medium at pH 6.3 containing 1.0 w/w% of yeast extract and 2.0 w/w% of aqueous glucose followed by starting pre-culturing (first stage) under conditions of reciprocally shaking at 100 rpm at a temperature of 28°C, and culturing for 3 days. Next, 30 L of a medium having a pH of 6.3 and containing 1.0 w/w% of yeast extract, 2.0 w/w% of aqueous glucose and 0.1 w/w% of soybean oil was prepared in a 50 L fermentation vessel, and 200 mL of the seed culture broth (first stage) was inoculated therein, followed by starting pre-culturing (second stage) and culturing for 2 days under conditions of a temperature of 28°C. Next, 25 L of the seed culture broth (second stage) was inoculated into a main culture initial medium (5.0 w/w% of defatted soybean powder, 0.3 w/w% of K₂HPO₄, 0.05 w/w% of MgCl₂·6H₂O, 0.05 w/w% of CaCl₂·2H₂0, pH 6.0), and brought to a total of 4000 L for the amount of the initial culture medium.

Culturing was started at a temperature of 26°C and internal pressure of 0.10 MPa. Aqueous glucose was added at 5.33% on day 1 of culturing, at 5.33% on day 2, at 5.33% on day 3, at 4.00% on day 4 and 4.00% on day 5, and as a result of adding aqueous glucose at a total of 24.0% in the same manner as <1>, glucose in the medium was completely depleted on day 9 of culturing. The amount of (5Z,8Z,11Z,14Z)-5,8,11,14- icosatetraenoic acid produced on day 12 of culturing was 18.3 g/L, and the ratio of (5Z,8Z,11Z,14Z)-5,8,11,14- icosatetraenoic acid to the total amount of fatty acid was 45.6%. Changes in the glucose concentration in the medium during fed-batch culturing in the case of initially adding glucose as in <1> and not initially adding glucose as in <2> are shown in FIG. 1, while changes in the ratio of (5Z,8Z,11Z,14Z)-5,8,11,14-icosatetraenoic acid to the total amount of fatty acid are shown in FIG. 2.

### Example 2

### <1> Production of (8Z,11Z,14Z)-8,11,14-icosatrienoic Acid Using Culturing System in which Glucose was Added to Initial Medium

*Mortierella alpina* strain S14 was used. Approximately one loopful of *Mortierella alpina* strain S14 was inoculated into 100 mL of medium at pH 6.3 containing 1.0 w/w% of yeast extract and 2.0 w/w% of aqueous glucose followed by starting pre-culturing (first stage) under conditions of reciprocally shaking at 100 rpm at a temperature of 28°C, and culturing for 3 days. Next, 30 L of a medium having a pH of 6.3 and containing 1.0 w/w% of yeast extract, 2.0 w/w% of aqueous glucose and 0.1 w/w% of soybean oil was prepared in a 50 L fermentation vessel, and 200 mL of the seed culture broth (first stage) was inoculated therein, followed by starting pre-culturing (second stage) and culturing for 2 days under conditions of a temperature of 28°C. Next, 25 L of the seed culture broth (second stage) was inoculated into a main culture initial medium (2.00 w/w% of aqueous glucose, 4.32 w/w% of defatted soybean powder, 0.3 w/w% of K₂HPO₄, 0.05 w/w% of MgSO₄·7H₂O, 0.05 w/w% of CaSO₄·2H₂0, pH 6.3), and brought to a total of 4000 L for the amount of the initial culture medium.

Culturing was started at a temperature of 26°C and internal pressure of 0.10 MPa. As a result of adding aqueous glucose of 4.00% on day 1 of culturing, 5.00% on day 2, 6.00% on day 3 and 6.00% on day 6 so as to add aqueous glucose to a total of 23.0%, and further adding 1.00% of disodium succinate and 0.021% of NaOH on day 4 of culturing, the concentration of residual glucose in the medium on day 13 of culturing was 0.75%.

### <2> Production of (8Z,11Z,14Z)-8,11,14-icosatrienoic Acid Using Culturing System in which Glucose was not dded to Initial Medium

*Mortierella alpina* strain S14 was used. Approximately one loopful of *Mortierella alpina* strain S14 was inoculated into 100 mL of medium at pH 6.3 containing 1.0 w/w% of yeast extract and 2.0 w/w% of aqueous glucose, followed by starting pre-culturing (first stage) under conditions of reciprocally shaking at 100 rpm at a temperature of 28°C, and culturing for 3 days. Next, 30 L of a medium having a pH of 6.3 and containing 1.0 w/w% of yeast extract, 2.0 w/w% of aqueous glucose and 0.1 w/w% of soybean oil was prepared in a 50 L fermentation vessel, and 200 mL of the seed culture broth (first stage) was inoculated therein, followed by starting pre-culturing (second stage) and culturing for 2 days under conditions of a temperature of 28°C. Next, 25 L of the seed culture broth (second stage) was inoculated into a main culture initial medium (4.32 w/w% of defatted soybean powder, 0.3 w/w% of K₂HPO₄, 0.05 w/w% of MgSO₄·7H₂O, 0.05 w/w% of CaSO₄·2H₂0, pH 6.3), and brought to a total of 4000 L for the amount of the initial culture medium.

Culturing was started at a temperature of 26°C and internal pressure of 0.10 MPa. As a result of adding aqueous glucose of 6.00% on day 1 of culturing, 5.00% on day 2, 6.00% on day 3 and 6.00% on day 6 so as to add aqueous glucose at a total of 23.0%, and further adding 1.00% of disodium succinate and 0.021% of NaOH on day 4 of culturing, glucose in the medium was completely depleted on day 12 of culturing.

### Example 3

### <1> Production of (5Z,8Z,11Z)-8,11,14-icosatrienoic Acid by Culturing with Glucose Added to Initial Medium

*Mortierella alpina* strain JT180 was used. Approximately one loopful of *Mortierella alpina* strain JT180 was inoculated into 20 mL of a medium at pH 6.0 containing 1.0 w/w% of yeast extract and 2.0 w/w% of aqueous glucose, followed by starting pre-culturing under conditions of reciprocally shaking at 100 rpm at a temperature of 28°C, and culturing for 3 days. Next, 20 ml of the pre-culture broth was inoculated into a main culture initial medium (2.00 w/w% of aqueous glucose, 1.00 w/w% of yeast extract, pH 6.0), and brought to a total of 5 L for the amount of the initial culture medium. After culturing for 2 days at a temperature of 28°C, the culturing temperature was changed to 19°C and culturing was continued. As a result of culturing by adding 1.00% of glucose on each of days 1 and 2 of culturing to add glucose at a total of 4.0%, glucose in the medium was completely depleted on day 6 of culturing.

### <2> Production of (5Z,8Z,11Z)-8,11,14-icosatrienoic Acid Using Culturing System in which Glucose was not Added to Initial Medium

*Mortierella alpina* strain JT180 was used. Approximately one loopful of *Mortierella alpina* strain JT180 was inoculated into 20 mL of a medium at pH 6.0 containing 1.0 w/w% of yeast extract and 2.0 w/w% of aqueous glucose, followed by starting pre-culturing under conditions of reciprocally shaking at 100 rpm at a temperature of 28°C, and culturing for 3 days. Next, 20 ml of the pre-culture broth was inoculated into a main culture initial medium (2.00 w/w% of aqueous glucose, 1.00 w/w% of yeast extract, pH 6.0), and brought to a total of 5 L for the amount of the initial culture medium. After culturing for 2 days at a temperature of 28°C, the culturing temperature was changed to 19°C and culturing was continued. When culturing was carried out by adding 1.00% of glucose on each of days 1 and 2 of culturing to add glucose at a total of 4.0%, glucose in the medium was completely depleted on day 5 of culturing.

According to the aforementioned results, the speed at which microorganisms consume carbohydrates and/or hydrocarbon oxidation products can be increased, the culturing period required to produce a useful substance can be shortened, and the productivity of a useful substance was determined to improve as a result of culturing microorganisms without adding carbohydrates and/or hydrocarbon oxidation products to the initial medium.

## Claims

1. A process for producing a useful substance by culturing microorganisms, comprising:
a pre-culturing step for pre-culturing the microorganisms,
a step for inoculating the resulting pre-culture broth into a main culture initial medium, wherein the total concentration of carbohydrate and hydrocarbon oxidation product in the main culture initial medium is less than 0.4% by weight in terms of the carbon equivalent, and
a main culturing step for adding carbohydrate and/or hydrocarbon oxidation product after the microorganisms have begun logarithmic growth.

2. The process for production according to claim 1, wherein the carbohydrate and/or hydrocarbon oxidation product is substantially not added to the main culture initial medium.

3. The process for production according to claim 1 or 2, wherein the carbohydrete and/or hydrocarbon oxidation product added after the microorganisms have begun logarithmic growth is a sugar.

4. The process for production according to any one of claims 1 to 3, wherein the microorganisms are microorganisms that catabolize the carbohydrate and/or hydrocarbon oxidation product with an exoamylase.

5. The process for production according to any one of claims 1 to 4, wherein the microorganisms are microorganisms belonging to the genus *Mortierella.*

6. The process for production according to any one of claims 1 to 5, wherein the useful substance is a highly unsaturated fatty acid.
